# EUROPÄISCHE PATENTSCHRIFT

(11) **EP 2 448 913 B1**
(45) Veröffentlichungstag und Bekanntmachung des Hinweises auf die Patenterteilung: **24.04.2013**
(21) Anmeldenummer: 10724308.1
(22) Anmeldetag: 09.06.2010
(51) Int. Cl.: C07C 231/02, C07C 233/47, C07C 233/53, C07C 303/22, C07C 309/14, B01J 19/12, H05B 6/70, H05B 6/80

(54) **KONTINUIERLICHES VERFAHREN ZUR ACYLIERUNG VON AMINOGRUPPEN TRAGENDEN ORGANISCHEN SÄUREN**
CONTINUOUS METHOD FOR ACYLATING AMINO GROUP-CARRYING ORGANIC ACIDS
PROCÉDÉ D'ACYLATION CONTINUE D'ACIDES ORGANIQUES PORTEURS DE GROUPES AMINO

(30) Priorität: 30.06.2009 DE 102009031056
(43) Veröffentlichungstag der Anmeldung: 09.05.2012
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE)
(74) Vertreter: Mikulecky, Klaus
(86) Internationale Anmeldenummer: PCT/EP2010/003444
(87) Internationale Veröffentlichungsnummer: WO 2011/000461

(56) Entgegenhaltungen:
- EP-A2- 1 491 552
- WO-A1-2008/043493
- DE-A1-102006 047 619

## Beschreibung

Die vorliegende Erfindung betrifft ein kontinuierliches Verfahren zur Acylierung von Aminogruppen tragenden organischen Säuren unter Mikrowellenbestrahlung im technischen Maßstab.

Acylierungsprodukte Aminogruppen tragender organischer Säuren finden als chemische Rohstoffe vielseitige Verwendung. So sind mit niederen Carbonsäuren N-acylierte Aminogruppen tragende organische Säuren insbesondere als Pharmazeutika bzw. als Zwischenprodukte für die Herstellung von Pharmazeutika interessant. Mit längerkettigen Fettsäuren N-acylierte Aminogruppen tragende organische Säuren haben amphiphile Eigenschaften, weshalb sie vielfältige Verwendung als Bestandteil in Wasch- und Reinigungsmitteln sowie in Kosmetika finden. Weiterhin werden sie erfolgreich als Hilfsmittel in der Metallbearbeitung, bei der Formulierung von Pflanzenschutzmitteln, als Antistatika für Polyolefine sowie bei der Förderung und Verarbeitung von Erdöl verwendet.

Bei der technischen Herstellung von N-Acylierungsprodukten Aminogruppen tragender Säuren wird üblicherweise ein reaktives Derivat einer Carbonsäure wie Säureanhydrid, Säurechlorid oder Ester mit der mindestens eine Aminogruppe tragenden Säure umgesetzt, wobei meistens in alkalischem Milieu gearbeitet wird. Dies führt einerseits zu hohen Herstellkosten und andererseits zu unerwünschten Begleitprodukten wie beispielsweise Salzen oder Säuren, die abgetrennt und entsorgt oder aufgearbeitet werden müssen. So entstehen beispielsweise in der Schotten-Baumann-Synthese, nach der zahlreiche Amide von Säuregruppen tragenden Aminen großtechnisch hergestellt werden, mindestens equimolare Mengen Kochsalz. Die ebenfalls praktizierte Verwendung von Kupplungsreagentien wie N,N'-Dicyclohexylcarbodiimid (DCC) ist teuer, erfordert auf Grund der Toxizität der Kupplungsreagentien und ihrer Umsetzungsprodukte besondere Maßnahmen zur Arbeitsplatzhygiene und führt ebenfalls zu großen Mengen an zu entsorgenden Nebenprodukten. Die erstrebenswerte direkte thermische Kondensation von Carbonsäure und mindestens eine Säuregruppen tragendem Amin erfordert sehr hohe Temperaturen und lange Reaktionszeiten, wobei jedoch nur moderate Ausbeuten erhalten werden (J. Am. Chem. Soc., 59 (1937), 401-402). Unter diesen Reaktionsbedingungen bereitet zudem die Korrosivität der Reaktionsgemische aus Säure, Amin, Amid und Reaktionswasser große technische Probleme, da diese Gemische bei den erforderlichen hohen Reaktionstemperaturen metallische Reaktionsgefäße stark angreifen bzw. auflösen. Die dadurch in die Produkte eingetragenen Metallgehalte sind sehr unerwünscht, da sie die Produkteigenschaften nicht nur hinsichtlich ihrer Farbe beeinträchtigen sondern auch Zersetzungsreaktionen katalysieren und damit die Ausbeute reduzieren. Umgangen werden kann letzteres Problem zum Teil durch spezielle Reaktionsgefäße aus hoch korrosionsbeständigen Materialien oder mit entsprechenden Beschichtungen, was aber trotzdem lange Reaktionszeiten erfordert und somit zu in ihrer Farbe beeinträchtigten Produkten führt. Weiterhin ist die Auftrennung von eingesetzter Carbonsäure und gebildetem Amid oftmals äußerst aufwendig, da beide häufig sehr ähnliche Siedepunkte besitzen und zudem Azeotrope bilden.

Ein neuerer Ansatz zur Synthese von Amiden ist die durch Mikrowellen unterstützte Umsetzung von Carbonsäuren und Aminen zu Amiden.

Vázquez-Tato, Synlett 1993, 506, offenbart die Verwendung von Mikrowellen als Heizquelle für die Herstellung von Amiden aus Carbonsäuren und arylaliphatischen Aminen über die Ammoniumsalze. Die Synthesen erfolgen im mmol-Maßstab.

Gelens et al., Tetrahedron Letters 2005, 46(21), 3751-3754, offenbaren eine Vielzahl an Amiden, die unter Zuhilfenahme von Mikrowellenstrahlung synthetisiert wurden. Die Synthesen erfolgen in 10 ml-Gefäßen. Säuregruppen tragende Amine werden nicht eingesetzt.

Das Scale-Up derartiger mikrowellenunterstützter Reaktionen aus dem Labor in einen technischen Maßstab und damit die Entwicklung von Anlagen, die für eine Produktion von mehreren Tonnen wie beispielsweise mehreren zehn, mehreren hundert oder mehreren tausend Tonnen pro Jahr mit für großtechnische Anwendungen interessanten Raum-Zeit-Ausbeuten geeignet sind, konnte bisher jedoch nicht realisiert werden. Ursache dafür ist zum einen die üblicherweise auf einige Millimeter bis wenige Zentimeter begrenzte Eindringtiefe von Mikrowellen in das Reaktionsgut, was insbesondere in Batch-Verfahren durchgeführte Reaktionen auf kleine Gefäße beschränkt oder in gerührten Reaktoren zu sehr langen Reaktionszeiten führt. Einer für die Bestrahlung großer Substanzmengen mit Mikrowellen wünschenswerten Erhöhung der Feldstärke sind insbesondere in den bisher bevorzugt zum Scale-Up chemischer Reaktionen eingesetzten Multimode-Geräten durch dann auftretende Entladungsvorgänge und Plasmenbildung enge Grenzen gesetzt. Weiterhin bereitet die in Multimode-Mikrowellengeräten zu lokalen Überhitzungen des Reaktionsgutes führende, durch mehr oder weniger unkontrollierte Reflektionen der in den Mikrowellenofen eingestrahlten Mikrowellen an dessen Wänden und dem Reaktionsgut verursachte Inhomogenität des Mikrowellenfeldes Probleme bei der Maßstabsvergrößerung. Zudem bereitet der sich während der Reaktion oftmals ändernde Mikrowellen-Absorptionskoeffizient des Reaktionsgemischs Schwierigkeiten hinsichtlich einer sicheren und reproduzierbaren Reaktionsführung.

Chen et al. (J. Chem. Soc., Chem. Commun., 1990, 807 - 809), beschreiben einen kontinuierlichen Labor-Mikrowellenreaktor, bei dem das Reaktionsgut durch eine in einem Mikrowellenofen montierte Teflon-Rohrschlange geführt wird. Ein ähnlicher kontinuierlicher Labor-Mikrowellenreaktor wird von Cablewski et al. (J. Org. Chem. 1994, 59, 3408-3412) zur Durchführung verschiedenster chemischer Reaktionen beschrieben. In beiden Fällen erlaubt die im Multimode betriebene Mikrowelle jedoch aus oben beschriebenen Gründen kein Up-Scaling in den großtechnischen Bereich. Zudem ist der Wirkungsgrad dieser Verfahren bezüglich der Mikrowellenabsorption des Reaktionsguts auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung kann zu unerwünschten Plasma- Entladungen oder zu so genannten thermischen Runaway-Effekten führen. Weiterhin machen die als Hot-Spots bezeichneten, sich zeitlich verändernden räumlichen Inhomogenitäten des Mikrowellenfeldes im Applikatorraum eine sichere und reproduzierbare Reaktionsführung im großen Maßstab unmöglich.

Weiterhin sind aus Kappe et al., Top. Curr. Chem. (2006) 266: 233 - 276, Monomode- bzw. Singlemode-Mikrowellenapplikatoren bekannt, bei denen mit einem einzigen Mikrowellen-Modus gearbeitet wird, der sich in nur einer Raumrichtung ausbreitet und durch exakt dimensionierte Wellenleiter auf das Reaktionsgefäß fokussiert wird. Diese Geräte erlauben zwar höhere lokale Feldstärken, sind bisher aber auf Grund der geometrischen Anforderungen (z. B. ist die Intensität des elektrischen Feldes an seinen Wellenbergen am größten und geht an den Knotenpunkten gegen Null) bisher auf kleine Reaktionsvolumina (≤ 50 ml) im Labormaßstab beschränkt.

EP-A-1491552 beschreibt eine mikrowellenunterstützte Peptidsynthese durch die Reaktion von zwei Aminosäuren (vergl. Patentanspruch 1). Die bekannte Peptidsynthese kann wahlweise kontinuierlich oder in einem Batch durchgeführt werden. Es gibt aber keinerlei Offenbarung über die Ausrichtung der Mikrowellen.

WO-2008/043493 offenbart ein Verfahren zur Synthese von Fettsäurealkanolamiden durch kontinuierliche Amidierung von Fettsäuren unter Einsatz von Mikrowellen. Die Mikrowellen werden dabei in einer Ausführungsform mittels mindestens einer Antenne über die Rohrenden eingestrahlt. Den Unterschied des daraus bekannten Verfahrens gegenüber dem erfindungsgemäßen Verfahren besteht darin, dass statt der erfindungsgemäßen Aminosäure dort ein Aminoalkohol beschrieben ist.

DE-A-102006047619 beschreibt ein Verfahren zur Synthese von Aminogruppen tragenden Fettsäureamiden durch kontinuierliche Amidierung von Fettsäuren unter Einsatz von Mikrowellen. Die Mikrowellen werden dabei in einer Ausführungsform mittels Antennen über die Rohrenden eingestrahlt. Diese Lehre unterscheidet sich von dem erfindungsgemäßen Verfahren dadurch, dass dort statt der erfindungsgemäßen Aminosäure ein Diamin beschrieben ist.

Es wurde daher ein Verfahren zur Herstellung von N-Acylierungsprodukten von Aminogruppen tragenden organischen Säuren gesucht, bei dem Carbonsäure und Aminogruppe tragende organische Säure auch im technischen Maßstab unter Mikrowellenbestrahlung zum Amid umgesetzt werden können. Dabei sollen möglichst hohe, das heißt bis zu quantitative Umsetzungsraten und Ausbeuten erzielt werden. Das Verfahren soll weiterhin eine möglichst energiesparende Herstellung der Amide ermöglichen, das heißt, die eingesetzte Mikrowellenleistung soll möglichst quantitativ vom Reaktionsgut absorbiert werden und das Verfahren somit einen hohen energetischen Wirkungsgrad bieten. Dabei sollen keine bzw. nur untergeordnete Mengen an Nebenprodukten anfallen. Die Amide sollen ferner einen möglichst niedrigen Gehalt an katalytisch aktiven Metallionen insbesondere der Nebengruppenmetalle wie beispielsweise Eisen und eine geringe Eigenfärbung aufweisen. Zudem soll das Verfahren eine sichere und reproduzierbare Reaktionsführung gewährleisten.

Überraschenderweise wurde gefunden, dass sich N-Acylierungsprodukte von Aminogruppen tragenden organischen Säuren durch direkte Umsetzung von Carbonsäuren mit Aminogruppen tragenden organischen Säuren in einem kontinuierlichen Verfahren durch nur kurzzeitiges Erhitzen mittels Bestrahlung mit Mikrowellen in einem Reaktionsrohr, dessen Längsachse sich in der Ausbreitungsrichtung der Mikrowellen eines Monomode-Mikrowellenapplikators befindet, in technisch relevanten Mengen herstellen lassen. Dabei wird die in den Mikrowellenapplikator eingestrahlte Mikrowellenenergie praktisch quantitativ vom Reaktionsgut absorbiert. Das erfindungsgemäße Verfahren besitzt zudem eine hohe Sicherheit bei der Durchführung und bietet eine hohe Reproduzierbarkeit der eingestellten Reaktionsbedingungen. Die nach dem erfindungsgemäßen Verfahren hergestellten N-Acylierungsprodukte Säuregruppen tragender organischer Säuren zeigen eine im Vergleich zu nach konventionellen Herstellverfahren ohne zusätzliche Verfahrensschritte nicht zugängliche hohe Reinheit und niedrige Eigenfärbung.

Gegenstand der Erfindung ist ein kontinuierliches Verfahren zur N-Acylierung von Aminogruppen tragenden organischen Säuren, in dem mindestens eine Carbonsäure der Formel (I)

R¹-COOH (I)

worin R¹ für Wasserstoff oder einen gegebenenfalls substituierten Kohlenwasserstoffrest mit 1 bis 50 Kohlenstoffatomen steht,
mit mindestens einer mindestens eine Aminogruppe tragenden organischen Säure der Formel (II)

R²NH-A-X (II)

worin
- A: für einen gegebenenfalls substituierten Kohlenwasserstoffrest mit 1 bis 50 Kohlenstoffatomen,
- X: für eine Säuregruppe oder deren Metallsalz und
- R²: für Wasserstoff, einen gegebenenfalls substituierten Kohlenwasserstoffrest mit 1 bis 50 C-Atomen oder eine Gruppe der Formel -A-X, in der A wie auch X unabhängig voneinander die oben angegebenen Bedeutungen haben, stehen,

unter Mikrowellenbestrahlung in einem Reaktionsrohr, dessen Längsachse sich in der Ausbreitungsrichtung der Mikrowellen eines Monomode-Mikrowellenapplikators befindet, zum Amid umgesetzt wird, bei dem die Bestrahlung des Reaktionsgemischs mit Mikrowellen in einem weitgehend mikrowellentransparenten Reaktionsrohr innerhalb eines mit einem Mikrowellengenerator über Wellenleiter verbundenen Hohlleiters erfolgt.

Als Carbonsäuren der Formel I sind allgemein Verbindungen geeignet, die mindestens eine Carboxylgruppe an einem gegebenenfalls substituierten Kohlenwasserstoffrest mit 1 bis 50 C-Atomen besitzen sowie Ameisensäure. Der Kohlenwasserstoffrest kann aliphatisch oder aromatisch sein.

In einer ersten bevorzugten Ausführungsform ist der Kohlenwasserstoffrest R¹ ein aliphatischer, unsubstituierter Alkyl- oder Alkenylrest. In einer weiteren bevorzugten Ausführungsform trägt der aliphatische Kohlenwasserstoffrest einen oder mehrere wie beispielsweise zwei, drei, vier oder mehr weitere Substituenten. Geeignete Substituenten sind beispielsweise Halogenatome, halogenierte Alkylreste, C₁-C₅-Alkoxy- wie beispielsweise Methoxy-, Poly(C₁-C₅-Alkoxy)-, Poly(C₁-C₅-Alkoxy)alkyl-, Carboxyl-, Amid-, Cyano-, Nitril-, Nitro- und/oder Arylgruppen mit 5 bis 20 Kohlenstoffatomen wie beispielsweise Phenylgruppen, mit der Maßgabe, dass diese Substituenten unter den Reaktionsbedingungen stabil sind und keine Nebenreaktionen wie beispielsweise Eliminierungsreaktionen eingehen. Die C₅-C₂₀-Arylgruppen können ihrerseits wiederum Substituenten wie beispielsweise Halogenatome, halogenierte Alkylreste, C₁-C₂₀-Alkyl-, C₂-C₂₀-Alkenyl-, C₁-C₅-Alkoxy- wie beispielsweise Methoxy-, Ester-, Amid-, Cyano-, Nitril-, und/oder Nitrogruppen tragen. Der aliphatische Kohlenwasserstoffrest trägt jedoch höchstens so viele Substituenten wie er Valenzen hat. In einer speziellen Ausführungsform trägt der aliphatische Kohlenwasserstoffrest R¹ eine oder mehrere weitere Carboxylgruppen. So ist das erfindungsgemäße Verfahren ebenso zur N-Acylierung von Aminogruppen tragenden organischen Säuren mit Polycarbonsäuren geeignet, die beispielsweise zwei, drei, vier oder mehr Carboxylgruppen tragen. Dabei können die Carboxylgruppen der Polycarbonsäure (I) vollständig oder auch nur teilweise amidiert werden. Der Amidierungsgrad lässt sich beispielsweise durch die Stöchiometrie zwischen Carbonsäure (I) und Aminogruppen tragender organischer Säure (II) im Reaktionsgemisch einstellen. Weiterhin bevorzugt trägt der aliphatische Kohlenwasserstoffrest R¹ keine Aminogruppen.

Erfindungsgemäß besonders bevorzugt sind Carbonsäuren (I), die einen aliphatischen Kohlenwasserstoffrest mit 1 bis 30 C-Atomen und insbesondere mit 2 bis 24 C-Atomen wie beispielsweise mit 3 bis 20 C-Atomen tragen. Sie können natürlichen oder synthetischen Ursprungs sein. Der aliphatische Kohlenwasserstoffrest kann auch Heteroatome wie beispielsweise Sauerstoff, Stickstoff, Phosphor und/oder Schwefel enthalten, bevorzugt jedoch nicht mehr als ein Heteroatom pro 3 C-Atome. Die aliphatischen Kohlenwasserstoffreste können linear, verzweigt oder zyklisch sein. Die Carboxylgruppe kann an einem primären, sekundären oder tertiären C-Atom gebunden sein. Bevorzugt ist sie an einem primären C-Atom gebunden. Die Kohlenwasserstoffreste können gesättigt oder, sofern ihr Kohlenwasserstoffrest R¹ mindestens 2 Kohlenstoffatome umfasst, auch ungesättigt sein. Ungesättigte Kohlenwasserstoffreste enthalten bevorzugt eine oder mehrere C=C-Doppelbindungen und besonders bevorzugt eine, zwei oder drei C=C-Doppelbindungen. So hat sich das erfindungsgemäße Verfahren besonders zur Herstellung von Amiden mehrfach ungesättigter Carbonsäuren bewährt, da die Doppelbindungen der ungesättigten Carbonsäuren unter den Reaktionsbedingungen des erfindungsgemäßen Verfahrens nicht angegriffen werden. Bevorzugte zyklische aliphatische Kohlenwasserstoffreste besitzen mindestens einen Ring mit vier, fünf, sechs, sieben, acht oder mehr Ringatomen.

In einer bevorzugten Ausführungsform steht R¹ für einen gesättigten Alkylrest mit 1, 2, 3 oder 4 C-Atomen. Dieser kann linear oder auch verzweigt sein. Die Carboxylgruppe kann an einem primären, sekundären oder, wie im Falle der Pivalinsäure, tertiären C-Atom gebunden sein. In einer besonders bevorzugten Ausführungsform ist der Alkylrest ein unsubstituierter Alkylrest. In einer weiteren besonders bevorzugten Ausführungsform trägt der Alkylrest einen bis neun, bevorzugt einen bis fünf wie beispielsweise zwei, drei oder vier weitere Substituenten. Bevorzugte weitere Substituenten sind Carboxylgruppen sowie gegebenenfalls substituierte C₅-C₂₀-Arylreste.

In einer weiteren bevorzugten Ausführungsform handelt es sich bei der Carbonsäure (I) um eine ethylenisch ungesättigte Carbonsäure. Dabei steht R¹ für eine gegebenenfalls substituierte Alkenylgruppe mit 2 bis 4 Kohlenstoffatomen. Unter ethylenisch ungesättigten Carbonsäuren werden hier solche Carbonsäuren verstanden, die eine zur Carboxylgruppe konjugierte C=C-Doppelbindung besitzen. Die Alkenylgruppe kann linear oder, sofern sie mindestens drei C-Atome umfasst, verzweigt sein. In einer bevorzugten Ausführungsform ist der Alkenylrest ein unsubstituierter Alkenylrest. Besonders bevorzugt ist R¹ ein Alkenylrest mit 2 oder 3 C-Atomen. In einer weiteren bevorzugten Ausführungsform trägt der Alkenylrest einen oder mehrere, wie beispielsweise zwei, drei oder mehr weitere Substituenten. Der Alkenylrest trägt jedoch höchstens so viele Substituenten, wie er Valenzen hat. In besonders bevorzugten Ausführungsformen trägt der Alkenylrest R¹ als weiteren Substituenten eine Carboxylgruppe oder eine gegebenenfalls substituierte C₅-C₂₀-Arylgruppe. So ist das erfindungsgemäße Verfahren ebenso zur Umsetzung von ethylenisch ungesättigten Dicarbonsäuren geeignet.

In einer weiteren bevorzugten Ausführungsform handelt es sich bei der Carbonsäure (I) um eine Fettsäure. Dabei steht R¹ für einen gegebenenfalls substituierten aliphatischen Kohlenwasserstoffrest mit 5 bis 50 C-Atomen. Besonders bevorzugt sind dabei Fettsäuren, die einen aliphatischen Kohlenwasserstoffrest mit 6 bis 30 C-Atomen und insbesondere mit 7 bis 26 C-Atomen wie beispielsweise mit 8 bis 22 C-Atomen tragen. In einer bevorzugten Ausführungsform ist der Kohlenwasserstoffrest der Fettsäure ein unsubstituierter Alkyl- oder Alkenylrest. In einer weiteren bevorzugten Ausführungsform trägt der Kohlenwasserstoffrest der Fettsäure einen oder mehrere wie beispielsweise zwei, drei, vier oder mehr weitere Substituenten. In einer speziellen Ausführungsform trägt der Kohlenwasserstoffrest der Fettsäure eine, zwei, drei, vier oder mehr weitere Carboxylgruppen.

In einer weiteren bevorzugten Ausführungsform ist der Kohlenwasserstoffrest R¹ ein aromatischer Rest. Unter aromatischen Carbonsäuren (I) werden hier allgemein Verbindungen verstanden, die mindestens eine an ein aromatisches System gebundene Carboxylgruppe tragen. Unter aromatischen Systemen werden zyklische, durchkonjugierte Systeme mit (4n + 2) π-Elektronen verstanden, worin n eine natürliche ganze Zahl und vorzugsweise 1, 2, 3, 4 oder 5 ist. Das aromatische System kann mono- oder polyzyklisch wie beispielsweise di- oder trizyklisch sein. Das aromatische System wird bevorzugt aus Kohlenstoffatomen gebildet. In einer weiteren bevorzugten Ausführungsform enthält es neben Kohlenstoffatomen ein oder mehrere Heteroatome wie beispielsweise Stickstoff, Sauerstoff und/oder Schwefel. Beispiele für solche aromatischen Systeme sind Benzol, Naphthalin, Phenanthren, Indol, Furan, Pyridin, Pyrrol, Thiopen und Thiazol. Das aromatische System kann neben der Carboxylgruppe einen oder mehrere wie beispielsweise einen, zwei, drei oder mehr gleiche oder verschiedene weitere Substituenten tragen. Geeignete weitere Substituenten sind beispielsweise Halogenatome, Alkyl- und Alkenylreste sowie Hydroxy-, Hydroxyalkyl-, Alkoxy-, Poly(alkoxy)-, Amid-, Cyano- und/oder Nitrilgruppen. Diese Substituenten können an beliebiger Position des aromatischen Systems gebunden sein. Der Arylrest trägt jedoch höchstens so viele Substituenten, wie er Valenzen hat. Bevorzugt trägt der Arylrest keine Aminogruppen.

In einer speziellen Ausführungsform trägt der Arylrest der aromatischen Carbonsäure (I) weitere Carboxylgruppen. So ist das erfindungsgemäße Verfahren ebenso zur Umsetzung von aromatischen Carbonsäuren mit beispielsweise zwei oder mehr Carbonsäuregruppen geeignet. Die Carbonsäuregruppen können gemäß dem erfindungsgemäßen Verfahren vollständig oder auch nur teilweise in Amide überführt werden. Der Amidierungsgrad lässt sich beispielsweise durch die Stöchiometrie zwischen Carbonsäure und Aminogruppen tragender organischer Säure im Reaktionsgemisch einstellen.

Besonders geeignet ist das erfindungsgemäße Verfahren weiterhin zur Herstellung von Alkylarylcarbonsäureamiden wie beispielsweise Alkylphenylcarbonsäureamiden. Dabei werden nach dem erfindungsgemäßen Verfahren aromatische Carbonsäuren (I), bei denen der die Carbonsäuregruppe tragende Arylrest zusätzlich mindestens einen Alkyl- oder Alkylenrest trägt, mit Aminogruppen tragenden organischen Säuren (II) umgesetzt. Besonders vorteilhaft ist das Verfahren dabei zur Herstellung von Alkylbenzoesäureamiden, deren Arylrest mindestens einen Alkylrest mit 1 bis 20 C-Atomen und insbesondere 1 bis 12 C-Atomen wie beispielsweise 1 bis 4 C-Atomen trägt.

Weiterhin besonders geeignet ist das erfindungsgemäße Verfahren zur Herstellung aromatischer Carbonsäureamide, deren Arylrest R¹ eine oder mehrere wie beispielsweise zwei oder drei Hydroxylgruppen und/oder Hydroxyalkylgruppen trägt. Bei der Umsetzung der entsprechenden Carbonsäuren (I) insbesondere mit höchstens equimolaren Mengen an Aminogruppen tragenden organischen Säuren der Formel (II) findet dabei selektiv eine Amidierung der Carboxylgruppe und keine Aminolyse der phenolischen OH-Gruppe statt.

Zur Amidierung gemäß dem erfindungsgemäßen Verfahren geeignete Carbonsäuren (I) sind beispielsweise Ameisensäure, Essigsäure, Propionsäure, Buttersäure, iso-Buttersäure, Pentansäure, iso-Pentansäure, Pivalinsäure, Acrylsäure, Methacrylsäure, Crotonsäure, 2,2-Dimethylacrylsäure, Maleinsäure, Fumarsäure, Itaconsäure, Zimtsäure und Methoxyzimtsäure, Bernsteinsäure, Butantetracarbonsäure, Phenylessigsäure, (2-Bromphenyl)essigsäure, (Methoxyphenyl)essigsäure, (Dimethoxyphenyl)essigsäure, 2-Phenylpropionsäure, 3-Phenylpropionsäure, 3-(4-Hydroxyphenyl)propionsäure, 4-Hydroxyphenoxyessigsäure, Hexansäure, Cyclohexansäure, Heptansäure, Octansäure, Nonansäure, Neononansäure, Decansäure, Neodecansäure, Undecansäure, Neoundecansäure, Dodecansäure, Tridecansäure, iso-Tridecansäure, Tetradecansäure, 12-Methyltridecansäure, Pentadecansäure, 13-Methyltetradecansäure, 12-Methyltetradecansäure, Hexadecansäure, 14-Methylpentadecansäure, Heptadecansäure, 15-Methylhexadecansäure, 14-Methylhexadecansäure, Octadecansäure, Iso-Octadecansäure, Icosansäure, Docosansäure und Tetracosansäure, Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure, Dodecenylbernsteinsäure und Octadecenylbernsteinsäure und aus ungesättigten Fettsäuren herstellbare Dimerfettsäuren sowie deren Mischungen. Weiterhin geeignet sind aus natürlichen Fetten und Ölen wie beispielsweise Baumwollsamen-, Cocos-, Erdnuss-, Färberdistel-, Mais-, Palmkem-, Raps-, Oliven-, Senfsamen, Soja-, Sonnenblumenöl sowie Talg-, Knochen- und Fischöl gewonnene Carbonsäuremischungen. Als Carbonsäuren bzw. Carbonsäuremischungen für das erfindungsgemäße Verfahren ebenfalls geeignet sind Tallölfettsäure sowie Harz- und Naphthensäuren. Weitere zur Amidierung gemäß dem erfindungsgemäßen Verfahren geeignete Carbonsäuren (I) sind beispielsweise Benzoesäure, Phthalsäure, Isophthalsäure, die verschiedenen Isomeren der Naphthalincarbonsäure, Pyridincarbonsäure und Naphthalindicarbonsäure sowie von Trimellitsäure, Trimesinsäure, Pyromellitsäure und Mellitsäure, den verschiedenen Isomeren der Methoxybenzoesäure, Hydroxybenzoesäure, Hydroxymethylbenzoesäure, Hydroxymethoxybenzoesäure, Hydroxydimethoxybenzoesäure, Hydroxyisophthalsäure, Hydroxynaphthalincarbonsäure, Hydoxypyridincarbonsäure und Hydroxymethylpyridincarbonsäure, Hydroxychinolincarbonsäure sowie von o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure und 3,4-Dimethylbenzoesäure. Mischungen verschiedener Aryl- und/oder Alkylarylcarbonsäuren sind gleichfalls geeignet.

Die mindestens eine Aminogruppe tragende organische Säure (II) trägt mindestens eine über den gegebenenfalls substituierten Kohlenwasserstoffrest A an den Stickstoff der Aminogruppe gebundene saure Gruppe X. Unter sauren Gruppen X werden funktionelle Gruppen verstanden, die mindestens ein acides Proton abspalten können. Erfindungsgemäß bevorzugte saure Gruppen X sind Carbonsäuren und organische Säuren des Schwefels und Phosphors wie beispielsweise Sulfonsäuren und Phosphonsäuren.

Der Kohlenwasserstoffrest A steht bevorzugt für einen aliphatischen oder aromatischen Rest mit der Maßgabe, dass A nicht für eine Acylgruppe bzw. für einen über eine Acylgruppe an den Stickstoff gebundenen Kohlenwasserstoffrest steht.

In einer ersten bevorzugten Ausführungsform ist A ein aliphatischer Rest mit 1 bis 12 und besonders bevorzugt mit 2 bis 6 C-Atomen. Er kann linear, zyklisch und/oder oder verzweigt sein. Bevorzugt ist er gesättigt. A kann weitere Substituenten tragen. Geeignete weitere Substituenten sind beispielsweise Carbonsäureamide, Guanidinreste, gegebenenfalls substituierte C₆-C₁₂-Arylreste wie beispielsweise Indol und Imidazol, sowie Säuregruppen wie beispielsweise Carbonsäuren und/oder Phosphonsäuregruppen. Der Rest A kann auch Hydroxylgruppen tragen, wobei die Umsetzung in diesem Fall aber mit höchstens equimolaren Anteilen an Carbonsäuren (I) erfolgen muss, um eine Acylierung dieser OH-Gruppen zu vermeiden. In einer besonders bevorzugten Ausführungsform trägt der aliphatische Rest A die Säuregruppe X an dem zum Stickstoffatom α- oder β-ständigen C-Atom. Besonders bewährt hat sich das erfindungsgemäße Verfahren zur Acylierung von Aminogruppen tragenden aliphatischen Säuren, in denen A für einen Alkylrest mit 1 bis 12 C-Atomen steht und in der sich die Säuregruppe X an dem zum Stickstoffatom α- oder β-ständigen C-Atom befindet und insbesondere von α-Aminocarbonsäuren, β-Aminosulfonsäuren und Aminomethylenphosphonsäuren.

In einer weiteren bevorzugten Ausführungsform steht A für einen aromatischen Kohlenwasserstoffrest mit 5 bis 12 C-Atomen. Unter aromatischen Systemen werden hier zyklische, durchkonjugierte Systeme mit (4n + 2) π-Elektronen verstanden, worin n eine natürliche ganze Zahl und vorzugsweise 1, 2, 3, 4 oder 5 ist. Das aromatische System kann mono- oder polyzyklisch wie beispielsweise di- oder trizyklisch sein; bevorzugt ist es monozyklisch. Der aromatische Rest A kann ein oder mehrere Heteroatome wie beispielsweise Sauerstoff, Stickstoff und/oder Schwefel enthalten. Amino- und Säuregruppe dieser mindestens eine Aminogruppe tragenden aromatischen Säure (II) können ortho-, meta- wie auch paraständig am aromatischen System angeordnet sein und sich bei mehrkernigen aromatischen Systemen auch an verschiedenen Ringen befinden. Beispiele für geeignete aromatische Systeme A sind Benzol, Naphthalin, Phenanthren, Indol, Furan, Pyridin, Pyrrol, Thiophen und Thiazol. Weiterhin kann das aromatische System A neben Carboxyl- und Aminogruppe ein oder mehrere wie beispielsweise eins, zwei, drei oder mehr gleiche oder verschiedene weitere Substituenten tragen. Geeignete weitere Substituenten sind beispielsweise Halogenatome, Alkyl- und Alkenylreste sowie Hydroxyalkyl-, Alkoxy-, Poly(alkoxy)-, Amid-, Cyano- und/oder Nitrilgruppen. Diese Substituenten können an beliebiger Position des aromatischen Systems gebunden sein. Der Arylrest trägt jedoch höchstens so viele Substituenten, wie er Valenzen hat.

In einer bevorzugten Ausführungsform steht R² für einen aliphatischen Rest. Dieser hat bevorzugt 1 bis 24, besonders bevorzugt 2 bis 18 und speziell 3 bis 6 C-Atome. Der aliphatische Rest kann linear, verzweigt oder zyklisch sein. Er kann weiterhin gesättigt oder ungesättigt sein, bevorzugt ist er gesättigt. Der aliphatische Rest kann Substituenten wie beispielsweise Halogenatome, halogenierte Alkylreste, Hydroxyl-, C₁-C₅-Alkoxyalkyl-, Cyano-, Nitril-, Nitro- und/oder C₅-C₂₀-Arylgruppen wie beispielsweise Phenylreste tragen. Die C₅-C₂₀-Arylreste können ihrerseits gegebenenfalls mit Halogenatomen, halogenierten Alkylresten, Hydroxyl-, C₁-C₂₀-Alkyl-, C₂-C₂₀-Alkenyl-, C₁-C₅-Alkoxy- wie beispielsweise Methoxy-, Ester-, Amid-, Cyano-, und/oder Nitrilgruppen substituiert sein. Besonders bevorzugte aliphatische Reste R² sind Methyl, Ethyl, n-Propyl, iso-Propyl, n-Butyl, iso-Butyl und tert.-Butyl, n-Hexyl, Cyclohexyl, n-Octyl, n-Decyl, n-Dodecyl, Tridecyl, Isotridecyl, Tetradecyl, Hexadecyl, Octadecyl und Methylphenyl und speziell bevorzugt sind Methyl, Ethyl, Propyl und Butyl.

In einer weiteren bevorzugten Ausführungsform steht R² für eine gegebenenfalls substituierte C₆-C₁₂-Arylgruppe oder eine gegebenenfalls substituierte heteroaromatische Gruppe mit 5 bis 12 Ringgliedern. Bevorzugte Heteroatome sind Sauerstoff, Stickstoff und Schwefel. In einer speziellen Ausführungsform steht R² für eine weitere Gruppe der Formel -A-X, wobei sowohl A wie auch X unabhängig voneinander die oben gegebenen Bedeutungen haben.

Sofern die Kohlenwasserstoffreste A und/oder R² weitere Säuregruppen wie beispielsweise Carboxyl- und/oder Phosphonsäuregruppen tragen, sind Maßnahmen gegen die dabei zumindest partiell auftretende Polykondensation der mindestens eine Aminogruppe tragenden organischen Säure (II) zu treffen.

In einer besonders bevorzugten Ausführungsform steht R² für Wasserstoff.

Beispiele für erfindungsgemäß geeignete, mindestens eine Aminogruppe tragende organische Säuren (II) sind Aminosäuren wie Glycin, Alanin, Arginin, Asparagin, Glutamin, Histidin, Leucin, Isoleucin, Valin, Phenylalanin, Serin, Tyrosin, 3-Aminopropionsäure (β-Alanin), 3-Aminobuttersäure, 2-Aminobenzoesäure, 4-Aminobenzoesäure, 2-Aminoethansulfonsäure (Taurin), N-Methyltaurin, 2-(Aminomethyl)phosphonsäure, 1-Aminoethylphosphonsäure, 1-Amino-2-methylpropyl)phosphonsäure, (1-Amino-1-phosphono-octyl)-phosphonsäure.

Im erfindungsgemäßen Verfahren können Carbonsäure (I) und Aminogruppe tragende organische Säure (II) in beliebigen Verhältnissen miteinander zur Reaktion gebracht werden. Bevorzugt erfolgt die Umsetzung zwischen Carbonsäure (I) und Aminogruppe tragender organischer Säure (II) mit molaren Verhältnissen von 100:1 bis 1:10, bevorzugt von 10:1 bis 1:2, speziell von 3:1 bis 1:1,2, jeweils bezogen auf die Molequivalente an Carboxyl- in (I) und Aminogruppen in (II). In einer speziellen Ausführungsform werden Carbonsäure (I) und Aminogruppe tragende organische Säure (II) bezogen auf die Molequivalente an Carboxyl- in (I) und Aminogruppen in (II) equimolar eingesetzt.

In vielen Fällen hat es sich als vorteilhaft erwiesen, mit einem Überschuss an Carbonsäure (I), das heißt molaren Verhältnissen von Carboxyl- zu Aminogruppen von mindestens 1,01 : 1,00 und insbesondere zwischen 50 : 1 und 1,02 : 1 wie beispielsweise zwischen 10 : 1 und 1,1 : 1 zu arbeiten. Dabei werden die Aminogruppen praktisch quantitativ zum Amid umgesetzt. Besonders vorteilhaft ist dieses Verfahren, wenn die eingesetzte Carbonsäure leicht flüchtig ist. Leicht flüchtig heißt hier, dass die Carbonsäure (I) einen Siedepunkt bei Normaldruck von vorzugsweise unterhalb 200 °C wie beispielsweise unterhalb 160 °C besitzt und sich somit destillativ vom Amid abtrennen lässt.

Die erfindungsgemäße Herstellung der Amide erfolgt durch Umsetzung von Carbonsäure (I) und Aminogruppe tragender organische Säure (II) zum Ammoniumsalz und nachfolgende Bestrahlung des Salzes mit Mikrowellen in einem Reaktionsrohr, dessen Längsachse sich in der Ausbreitungsrichtung der Mikrowellen in einem Monomode-Mikrowellenapplikators befindet. Im einfachsten Falle gelingt die Umsetzung zum Ammoniumsalz durch Vermischen von Carbonsäure (I) und Aminogruppe tragender organischer Säure (II), gegebenenfalls in Gegenwart eines Lösemittels.

In vielen Fällen hat es sich ebenso bewährt, die mindestens eine Aminogruppe tragende organische Säure (II) vor der Umsetzung in ein Metallsalz zu überführen oder sie in Form eines Metallsalzes zur Umsetzung mit der Carbonsäure (I) einzusetzen. Des gleichen kann das Gemisch aus (I) und (II) mit einer bezogen auf die Konzentration der Säuregruppen X im wesentlichen equimolaren Menge an Base versetzt werden. Dafür bevorzugte Basen sind insbesondere anorganische Basen wie beispielsweise Metallhydroxide, -oxide, -carbonate, -silikate oder -alkoxide. Besonders bevorzugt sind dabei die Hydroxide, Oxide, Carbonate, Silikate oder Alkoxide von Alkali- oder Erdalkalimetallen wie beispielsweise Lithiumhydroxid, Natriumhydroxid, Kaliumhydroxid, Natriummethoxid, Kaliummethoxid, Natrium-tert.-butanolat, Kalium-tert.-butanolat, Natriumcarbonat und Kaliumcarbonat. In einer bevorzugten Ausführungsform erfolgt die Umsetzung zum Ammoniumsalz durch Zugabe einer Lösung der entsprechenden Base zum Beispiel in einem niederen Alkohol wie beispielsweise Methanol, Ethanol oder Propanol oder auch in Wasser zu einem der Reaktanden oder dem Reaktionsgemischs. Diese Arbeitsweise hat sich insbesondere bei der Acylierung von starken Säuregruppen X tragenden Aminen (II) wie beispielsweise Sulfon- oder Phosphonsäuregruppen tragenden Aminen (II) bewährt. Unter starken Säuren werden hier insbesondere Säuren mit einem pKs-Wert von unter 3,5 und speziell unter 3,0 verstanden.

In einer bevorzugten Ausführungsform wird zur Beschleunigung bzw. zur Vervollständigung der Reaktion in Gegenwart mindestens eines Katalysators gearbeitet. Vorzugsweise arbeitet man dabei in Gegenwart eines basischen Katalysators oder Gemischen aus mehreren dieser Katalysatoren. Als basische Katalysatoren werden im Rahmen der vorliegenden Erfindung ganz allgemein solche basischen Verbindungen eingesetzt, die geeignet sind, die Amidierung von Carbonsäuren mit Aminen zu Carbonsäureamiden zu beschleunigen. Diese Substanzen können in fester Form wie beispielsweise als Dispersion oder Festbett oder als Lösung wie beispielsweise als wässrige oder vorzugsweise als alkoholische Lösung eingesetzt werden. Beispiele geeigneter Katalysatoren sind anorganische und organische Basen wie beispielsweise Metallhydroxide, - oxide, carbonate, silikate oder -alkoxide. In einer bevorzugten Ausführungsform wird der basische Katalysator ausgewählt aus der Gruppe der Hydroxide, Oxide, Carbonate, Silikate oder Alkoxide von Alkali- oder Erdalkalimetallen. Dabei sind Lithiumhydroxid, Natriumhydroxid, Kaliumhydroxid, Natriummethoxid, Kaliummethoxid, Natrium-tert.-butanolat, Kalium-tert.-butanolat, Natriumcarbonat und Kaliumcarbonat ganz besonders bevorzugt. Auch Cyanidionen sind als Katalysator geeignet. Des Weiteren sind stark basische Ionenaustauscher als Katalysatoren geeignet. Die Menge der eingesetzten Katalysatoren hängt dabei von der Aktivität und Stabilität des Katalysators bei den gewählten Reaktionsbedingungen ab und ist der jeweiligen Reaktion anzupassen. Die Menge des einzusetzenden Katalysators kann dabei in weiten Grenzen variieren. Oftmals hat es sich bewährt, mit 0,1 bis 2,0 mol Base wie beispielsweise mit 0,2 bis 1,0 mol Base pro Mol eingesetztem Amin zu arbeiten. Besonders bevorzugt werden katalytische Mengen der oben genannten, reaktionsbeschleunigend wirkenden Verbindungen eingesetzt, bevorzugt im Bereich zwischen 0,001 und 10 Gew.-%, besonders bevorzugt im Bereich von 0,01 bis 5 Gew.-% wie beispielsweise zwischen 0,02 und 2 Gew.-%, bezogen auf die eingesetzte Menge an Carbonsäure (I) und Aminogruppe tragende Säure (II).

Die Bestrahlung des Reaktionsgemisches erfolgt mit Mikrowellen in einem weitgehend mikrowellentransparenten Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Weiterhin bevorzugt werden die im Hohlleiter nicht absorbierten Mikrowellen an seinem Ende reflektiert. Bevorzugt wird die Länge des Hohlraumresonators so dimensioniert, dass sich in ihm eine stehende Welle ausbildet. Durch Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Durch Verwendung eines Hohlraumresonators mit einer Länge, bei der n eine ganze Zahl ist, wird die Ausbildung einer stehenden Welle ermöglicht. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 3 bis 50 speziell von 4 bis 20 wie beispielsweise drei, vier, fünf, sechs, sieben, acht, neun oder zehn.

Die E₀₁ₙ-Mode des Hohlraumresonators wird in Englischer Sprache auch als TM₀₁ₙ-Mode bezeichnet, siehe beispielsweise K. Lange, K.H. Löcherer, Taschenbuch der Hochfrequenztechnik", Band 2, Seite K21 ff.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Reaktionsgemischs mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators. Dazu weist der Hohlraumresonator bevorzugt je eine zentrische Öffnung an zwei gegenüber liegenden Stirnwänden zum Hindurchführen des Reaktionsrohres auf.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Reaktionsgemisches mit Mikrowellen in einem mikrowellentransparenten Reaktionsrohr, das sich axialsymmetrisch in einem E₀₁ₙ-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Reaktionsgemisches mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich eine stehende Welle mit n = 2 oder mehr Feldmaxima der Mikrowelle ausbildet. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich eine stehende Welle mit n = 2 oder mehr Feldmaxima der Mikrowelle ausbildet.

Mikrowellengeneratoren, wie beispielsweise das Magnetron, das Klystron und das Gyrotron sind dem Fachmann bekannt.

Die zur Durchführung des erfindungsgemäßen Verfahrens eingesetzten Reaktionsrohre sind bevorzugt aus weitgehend mikrowellentransparentem, hoch schmelzendem Material gefertigt. Besonders bevorzugt werden nichtmetallische Reaktionsrohre eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε"/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Saphir, Zirkonoxid, Siliziumnitrid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Für das erfindungsgemäße Verfahren besonders geeignete Reaktionsrohre haben einen Innendurchmesser von einem Millimeter bis 50 cm, insbesondere zwischen 2 mm und 35 cm und speziell zwischen 5 mm und 15 cm wie beispielsweise zwischen 10 mm und 7 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Reaktionsrohres verstanden, auf der die Mikrowellenbestrahlung erfolgt. In das Reaktionsrohr können Stromstörer und/oder andere Mischelemente eingebaut sein.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Das Reaktionsrohr ist üblicherweise am Einlass mit einer Dosierpumpe sowie einem Manometer und am Auslass mit einer Druckhaltevorrichtung und einem Wärmetauscher versehen. Damit sind Reaktionen in einem sehr weiten Druck- und Temperaturbereich möglich.

Die Herstellung des Reaktionsgemischs aus Carbonsäure (I), der mindestens eine Aminogruppe tragenden organischen Säure (II) bzw. ihres Salzes und gegebenenfalls Katalysator kann kontinuierlich, diskontinuierlich oder auch in semi-Batch-Prozessen durchgeführt werden. So kann die Herstellung des Reaktionsgemischs in einem vorgelagerten (semi)-Batch Prozess durchgeführt werden wie beispielsweise in einem Rührbehälter. In einer bevorzugten Ausführungsform werden die Edukte Carbonsäure (I) und Aminogruppe tragende organische Säure (II) bzw. ihr Salz sowie gegebenenfalls der Katalysator, unabhängig voneinander gegebenenfalls mit Lösemittel verdünnt, erst kurz vor dem Eintritt in das Reaktionsrohr vermischt. Der Katalysator kann als solches oder als Mischung mit einem der Edukte dem Reaktionsgemisch zugefügt werden. So hat es sich besonders bewährt, die Mischung aus Carbonsäure, Aminogruppe tragender organischer Säure und Katalysator in einer Mischstrecke vorzunehmen, aus der das Reaktionsgemisch in das Reaktionsrohr gefördert wird. Weiterhin bevorzugt werden die Edukte und Katalysator dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Der Katalysator wird einem der Edukte oder auch der Eduktmischung vor dem Eintritt in das Reaktionsrohr zugesetzt. Auch heterogene Systeme können nach dem erfindungsgemäßen Verfahren umgesetzt werden, wobei entsprechende technische Vorrichtungen zum Fördern des Reaktionsgutes erforderlich sind.

Das Reaktionsgemisch kann in das Reaktionsrohr entweder an dem durch das Innenleiterrohr geführte Ende eingespeist werden als auch an dem entgegen gesetzten Ende. Das Reaktionsgemisch kann folglich parallel oder antiparallel zur Ausbreitungsrichtung der Mikrowellen durch den Mikrowellenapplikator geführt werden.

Durch Variation von Rohrquerschnitt, Länge der Bestrahlungszone (hierunter wird die Strecke des Reaktionsrohres verstanden, in dem das Reaktionsgut Mikrowellenstrahlung ausgesetzt ist), Fließgeschwindigkeit, Geometrie des Hohlraumresonators sowie der eingestrahlten Mikrowellenleistung werden die Reaktionsbedingungen bevorzugt so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Das Reaktionsgut kann zur Vervollständigung der Reaktion, gegebenenfalls nach Zwischenkühlung, mehrfach das Reaktionsrohr durchlaufen. Bei langsamer verlaufenden Reaktionen hat es sich oftmals bewährt, das Reaktionsprodukt nach Verlassen des Reaktionsrohres noch eine gewisse Zeit bei Reaktionstemperatur zu halten. Vielfach hat sich bewährt, wenn das Reaktionsprodukt unmittelbar nach Verlassen des Reaktionsrohres z. B. durch Mantelkühlung oder Entspannung abgekühlt wird. Auch hat es sich bewährt, den Katalysator unmittelbar nach Verlassen des Reaktionsrohres zu deaktivieren. Dies kann beispielsweise durch Neutralisation oder bei heterogen katalysierten Reaktionen durch Filtration geschehen.

Bevorzugt wird der durch die Mikrowellenbestrahlung bedingte Temperaturanstieg beispielsweise durch Regelung der Mikrowellenintensität, der Durchflussgeschwindigkeit und/oder durch Kühlung des Reaktionsrohres, beispielsweise durch einen Stickstoffstrom, auf maximal 500 °C begrenzt. Besonders bewährt hat sich die Durchführung der Umsetzung bei Temperaturen zwischen 150 °C und maximal 400 °C und speziell zwischen 170 °C und maximal 300 °C wie beispielsweise bei Temperaturen zwischen 180 °C und 270 °C.

Die Dauer der Mikrowellenbestrahlung hängt von verschiedenen Faktoren wie beispielsweise der Geometrie des Reaktionsrohres, der eingestrahlten Mikrowellenenergie, der speziellen Reaktion und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut beim Verlassen des Hohlraumresonators die gewünschte Maximaltemperatur hat. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt.

Bevorzugt wird die Umsetzung bei Drücken zwischen 1 bar (Atmosphärendruck) und 500 bar, besonders bevorzugt zwischen 1,5 bar und 200 bar, insbesondere zwischen 3 bar und 150 bar und speziell zwischen 10 bar und 100 bar wie beispielsweise zwischen 15 und 50 bar durchgeführt. Besonders bewährt hat sich das Arbeiten unter erhöhtem Druck, wobei oberhalb der Siedetemperatur (bei Normaldruck) der Edukte, Produkte, des gegebenenfalls anwesenden Lösemittels und/oder oberhalb des während der Reaktion gebildeten Reaktionswassers gearbeitet wird. Besonders bevorzugt wird der Druck so hoch eingestellt, dass das Reaktionsgemisch während der Mikrowellenbestrahlung im flüssigen Zustand verbleibt und nicht siedet.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

Auch wenn die Edukte Carbonsäure (I) und Aminogruppe tragende Säure (II) oftmals zu gut handhabbaren Reaktionsgemischen führen hat es sich in vielen Fällen bewährt, in Gegenwart von Lösemitteln zu arbeiten um beispielsweise die Viskosität des Reaktionsmediums abzusenken und/oder das Reaktionsgemisch, insbesondere sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen.

Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, Shellsol^{®} AB, Solvesso^{®} 150, Solvesso^{®} 200, Exxsol^{®}, Isopa^{®} und Shellsol^{®}-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt.

In einer weiteren bevorzugten Ausführungsform wird das erfindungsgemäße Verfahren in Lösemitteln mit höheren ε"-Werten von beispielsweise 5 und höher wie insbesondere mit ε"-Werten von 10 und höher durchgeführt, die zudem oftmals ein überlegenes Löseverhalten für die Aminogruppen tragenden Säuren (II) zeigen. Diese Ausführungsform hat sich weiterhin insbesondere bei der Umsetzung von Reaktionsgemischen bewährt, die selbst, das heißt ohne Anwesenheit von Löse- und/oder Verdünnungsmitteln nur eine sehr geringe Mikrowellenabsorption zeigen. So hat sich diese Ausführungsform insbesondere bei Reaktionsgemischen bewährt, die einen dielektrischen Verlust ε" von weniger als 10 und vorzugsweise weniger als 1 aufweisen. Auch Mischungen von Lösemitteln mit unterschiedlichen ε"-Werten haben sich für die erfindungsgemäßen Umsetzungen bestens bewährt. Besonders bevorzugte Lösemittel sind niedere Alkohole mit 1 bis 5 C-Atomen wie beispielsweise Methanol, Ethanol, n-Propanol, iso-Propanol, n-Butanol, iso-Butanol, tert.-Butanol, die verschiedenen Isomere des Pentanols, Ethylenglykol, Glycerin sowie Wasser. Die durch die Lösemittelzugabe oftmals beobachtete beschleunigte Aufheizung des Reaktionsgemischs erfordert jedoch Maßnahmen zur Einhaltung der Maximaltemperatur.

Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird die Reaktion in Gegenwart polarer Lösemittel wie niederen Alkoholen mit 1 bis 5 C-Atomen oder auch Wasser durchgeführt.

In einer weiteren bevorzugten Ausführungsform werden dem Reaktionsgemisch in diesem unlösliche, stark Mikrowellen absorbierende Substanzen zugegeben. Diese führen zu einer starken lokalen Erhitzung des Reaktionsgemischs und in der Folge zu weiter beschleunigten Reaktionen. Ein geeigneter derartiger Wärmesammler ist beispielsweise Graphit.

Als Mikrowellen werden elektromagnetische Strahlen mit einer Wellenlänge zwischen etwa 1 cm und 1 m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche, medizinische, häusliche oder ähnliche Anwendungen freigegebenen Frequenzen verwendet wie beispielsweise mit Frequenzen von 915 MHz, 2,45 GHz, 5,8 GHz oder 24,12 GHz.

Die für die Durchführung des erfindungsgemäßen Verfahrens in den Hohlraumresonator einzustrahlende Mikrowellenleistung ist insbesondere abhängig von der angestrebten Reaktionstemperatur, aber auch von der Geometrie des Reaktionsrohrs und damit des Reaktionsvolumens sowie der Durchflussgeschwindigkeit des Reaktionsguts durch das Reaktionsrohr. Sie liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden.

In einer bevorzugten Ausführungsform wird die Reaktion in einem druckfesten, chemisch inerten Rohr durchgeführt, wobei das sich bildende Reaktionswasser sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser, überschüssigen Edukten sowie gegebenenfalls Lösemittel und/oder zur Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Filtration, Destillation, Strippen, Flashen und/oder Absorption abgetrennt.

Zur Erzielung besonders hoher Umsetzungsgrade hat es sich in vielen Fällen bewährt, das erhaltene Reaktionsprodukt nach Entfernen von Reaktionswasser sowie gegebenenfalls Austragen von Produkt und/oder Nebenprodukt erneut der Mikrowellenbestrahlung auszusetzen, wobei gegebenenfalls das Verhältnis der eingesetzten Reaktanden um verbrauchte oder unterschüssige Edukte zu ergänzen ist.

Die Vorteile des erfindungsgemäßen Verfahrens liegen in einer sehr gleichmäßigen Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang befindet. Dabei erlaubt das erfindungsgemäße Reaktordesign die Durchführung von Reaktionen auch bei sehr hohen Drücken und/oder Temperaturen. Durch Temperatur- und/oder Druckerhöhung wird eine deutliche Steigerung von Umsetzungsgrad und Ausbeute auch gegenüber bekannten Mikrowellenreaktoren beobachtet, ohne dass es dabei zu unerwünschten Nebenreaktionen und/oder Verfärbungen kommt. Überraschenderweise wird dabei ein sehr hoher Wirkungsgrad bei der Ausnutzung der in den Hohlraumresonator eingestrahlten Mikrowellenenergie erzielt, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Das erfindungsgemäße Verfahren erlaubt zudem eine kontrollierte, sichere und reproduzierbare Reaktionsführung. Da das Reaktionsgut im Reaktionsrohr parallel zur Ausbreitungsrichtung der Mikrowellen bewegt wird, werden bekannte Überhitzungsphänomene durch unkontrollierbare Feldverteilungen, die zu lokalen Überhitzungen durch wechselnde Intensitäten des Mikrowellenfeldes beispielsweise in Wellenbergen und Knotenpunkten führen, durch die Fließbewegung des Reaktionsgutes ausgeglichen. Die genannten Vorteile erlauben es auch, mit hohen Mikrowellenleistungen von mehr als 1 kW wie beispielsweise 2 bis 10 kW und insbesondere 5 bis 100 kW und teilweise auch höher zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei war es überraschend, dass trotz der im kontinuierlich durchflossenen Strömungsrohr nur sehr kurzen Verweildauer des Reaktionsgemischs im Mikrowellenfeld eine sehr weitgehende N-Acylierung mit Umsätzen im Allgemeinen von über 80 %, oftmals auch über 90 % wie beispielsweise über 95 % bezogen auf die im Unterschuss eingesetzte Komponente stattfindet, ohne Bildung nennenswerter Mengen an Nebenprodukten. Weiterhin überraschend war, dass sich die genannten Umsätze unter diesen Reaktionsbedingungen ohne Abtrennung des bei der Amidierung gebildeten Reaktionswassers wie auch in Gegenwart von polaren Lösemitteln wie Wasser und/oder Alkoholen erzielen lassen. Bei einer entsprechenden Umsetzung dieser Reaktionsgemische in einem Strömungsrohr gleicher Dimensionierung unter thermischer Mantelheizung werden zur Erzielung geeigneter Reaktionstemperaturen extrem hohe Wandtemperaturen benötigt, die zur Bildung undefinierter Polymere und gefärbter Spezies führten, aber bei gleichem Zeitintervall deutlich geringere N-Acylierung bewirken. Des Weiteren besitzen die nach dem erfindungsgemäßen Verfahren hergestellten Produkte sehr niedrige Metallgehalte ohne dass es einer weiteren Aufarbeitung der Rohprodukte bedarf. So liegen die Metallgehalte der nach dem erfindungsgemäßen Verfahren hergestellten Produkte bezogen auf Eisen als Hauptelement üblicherweise unterhalb 25 ppm bevorzugt unterhalb 15 ppm, speziell unterhalb 10 ppm wie beispielsweise zwischen 0,01 und 5 ppm Eisen.

Das erfindungsgemäße Verfahren erlaubt somit eine sehr schnelle, energiesparende und kostengünstige Herstellung von Amiden Aminogruppen tragender organischer Säuren in hohen Ausbeuten und mit hoher Reinheit in großtechnischen Mengen. Bei diesem Verfahren keine wesentlichen Mengen an Nebenprodukten an. Es werden keine unerwünschten Nebenreaktionen wie beispielsweise eine Oxidation des Amins oder eine Decarboxylierung der Carbonsäure beobachtet, die die Ausbeute an Zielprodukt herabsetzten würden.

Derartig schnelle und selektive Umsetzungen sind nach klassischen Methoden nicht zu erzielen und waren alleine durch Heizen auf hohe Temperaturen nicht zu erwarten.

### Beispiele

Die Umsetzungen der Reaktionsgemische unter Mikrowellenbestrahlung erfolgten in einem Keramikrohr (60 x 1 cm), das sich axialsymmetrisch in einem zylindrischen Hohlraumresonator (60 x 10 cm) befand. An einer der Stirnseiten des Hohlraumresonators verlief das Keramikrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode), in dem sich eine stehende Welle ausbildete.

Die Mikrowellenleistung wurde über die Versuchsdauer jeweils in der Art eingestellt, dass die gewünschte Temperatur des Reaktionsguts am Ende der Bestrahlungszone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Reaktionszone (etwa 15 cm Strecke in einer isolierten Edelstahlkapillare, Ø 1 cm) mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurückgespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet.

Mittels einer Hochdruckpumpe und eines geeigneten Druckentlastungsventils wurde die Reaktionsmischung im Reaktionsrohr unter einen solchen Arbeitsdruck gesetzt, der ausreichte, um alle Edukte und Produkte bzw. Kondensationsprodukte stets im flüssigen Zustand zu halten. Die aus Carbonsäure und Alkohol hergestellten Reaktionsmischungen wurden mit einer konstanten Flussrate durch das Reaktionsrohr gepumpt und die Verweilzeit in der Bestrahlungszone durch Modifizierung der Strömungsgeschwindigkeit eingestellt.

Die Analytik der Produkte erfolgte mittels ¹H-NMR-Spektroskopie bei 500 MHz in CDCl₃. Die Bestimmung von Eisengehalten erfolgte mittels Atomabsorptionsspektroskopie.

### Beispiel 1: Herstellung von N-Lauroyl-N-methyltaurat

In einem 10 I Büchi-Rührautoklaven mit Rührer, Innenthermometer und Druckausgleich wurden 1,6 kg Methyltaurin (10 mol) in 4 Liter eines Wasser Isopropanol-Gemisches (3:2 Volumenanteile) gelöst und mit 2,0 kg Laurinsäure (10 mol) versetzt.

Das so erhaltene Gemisch wurde bei einem Arbeitsdruck von 35 bar kontinuierlich mit 5 l/h durch das Reaktionsrohr gepumpt und einer Mikrowellenleistung von 2,2 kW ausgesetzt, von denen 94 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 255 °C.

Es wurde ein Umsatz von 83 % d. Th. erreicht. Das Reaktionsprodukt enthielt <5 ppm Eisen. Nach destillativer Abtrennung von Isopropanol wurde eine farblose, klare Flüssigkeit mit hoher Schaumbildungsneigung erhalten.

### Beispiel 2: Herstellung von N-Acetylglycin-Na-Salz

In einem 10 I Büchi-Rührautoklaven mit Rührer, Innenthermometer und Druckausgleich wurden in 2 Liter Wasser gelöste 1,0 kg Natriumglycinat (27 mol) mit 3,2 kg Essigsäure (107 mol) versetzt.

Das so erhaltene Gemisch wurde bei einem Arbeitsdruck von 30 bar kontinuierlich mit 5 l/h durch das Reaktionsrohr gepumpt und einer Mikrowellenleistung von 1,8 kW ausgesetzt, von denen 92 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 261 °C.

Es wurde ein Umsatz von 90 % d. Th. erreicht. Das Reaktionsprodukt enthielt < 5 ppm Eisen.

### Beispiel 3: Herstellung von N-Stearoylglycin-Na-Salz

In einem 10 I Büchi-Rührautoklaven mit Rührer, Innenthermometer und Druckausgleich wurden 1,2 kg Glycin-Na-Salz (12 mol) in 3,5 Liter eines Wasser Isopropanol-Gemisches (2:2 Volumenanteile) gelöst und mit 2,65 kg Stearinsäure (9,3 mol) versetzt.

Das so erhaltene Gemisch wurde bei einem Arbeitsdruck von 35 bar kontinuierlich mit 4 l/h durch das Reaktionsrohr gepumpt und einer Mikrowellenleistung von 2,6 kW ausgesetzt, von denen 90 % vom Reaktionsgut absorbiert wurden. Die Verweilzeit des Reaktionsgemischs in der Bestrahlungszone betrug ca. 42 Sekunden. Am Ende des Reaktionsrohres hatte das Reaktionsgemisch eine Temperatur von 267 °C.

Es wurde ein Umsatz von 79 % d. Th. erreicht. Das Reaktionsprodukt enthielt < 5 ppm Eisen.

### Beispiel 4: Herstellung von 4-(N-Cocoyl)amidobenzoesäure

In einem 10 I Büchi-Rührautoklaven mit Rührer, Innenthermometer und Druckausgleich wurden 1,45 kg 4-Aminobenzoesäure (10,5 mol) und 2,25 kg Cocosfettsäure (10,5 mol) in 5 l Isopropanol unter Erwärmen gelöst.
Das so erhaltene Gemisch wurde bei einem Arbeitsdruck von 35 bar kontinuierlich mit 3,5 l/h durch das Reaktionsrohr gepumpt und einer Mikrowellenleistung von 1,6 kW ausgesetzt, von denen 87 % vom Reaktionsgut absorbiert wurden. Die Verweilzeit des Reaktionsgemischs in der Bestrahlungszone betrug ca. 49 Sekunden. Am Ende des Reaktionsrohres hatte das Reaktionsgemisch eine Temperatur von 281 °C.

Es wurde ein Umsatz von 85 % d. Th. erreicht. Das Reaktionsprodukt enthielt <5 ppm Eisen.

## Patentansprüche

1. Kontinuierliches Verfahren zur N-Acylierung von Aminogruppen tragenden organischen Säuren, in dem mindestens eine Carbonsäure der Formel (I)
R¹-COOH (I)
worin R¹ für Wasserstoff oder einen gegebenenfalls substituierten Kohlenwasserstoffrest mit 1 bis 50 Kohlenstoffatomen steht,
mit mindestens einer mindestens eine Aminogruppe tragenden organischen Säure der Formel (II)
R²NH-A-X (II)
worin
A für einen gegebenenfalls substituierten Kohlenwasserstoffrest mit 1 bis 50 Kohlenstoffatomen,
X für eine Säuregruppe oder deren Metallsalz und
R² für Wasserstoff, einen gegebenenfalls substituierten Kohlenwasserstoffrest mit 1 bis 50 C-Atomen oder eine Gruppe der Formel -A-X, in der A wie auch X unabhängig voneinander die oben angegebenen Bedeutungen haben, stehen,
unter Mikrowellenbestrahlung in einem Reaktionsrohr, dessen Längsachse sich in der Ausbreitungsrichtung der Mikrowellen eines Monomode-Mikrowellenapplikators befindet, zum Amid umgesetzt wird, bei dem die Bestrahlung des Reaktionsgemischs mit Mikrowellen in einem weitgehend mikrowellentransparenten Reaktionsrohr innerhalb eines mit einem Mikrowellengenerator über Wellenleiter verbundenen Hohlleiters erfolgt.

2. Verfahren nach Anspruch 1, bei dem der Mikrowellenapplikator als Hohlraumresonator ausgestaltet ist.

3. Verfahren nach Anspruch 1 und/oder 2, bei dem der Mikrowellenapplikator als Hohlraumresonator vom Reflexionstyp ausgestaltet ist.

4. Verfahren nach einem oder mehreren der Ansprüche 1 bis 3, bei dem das Reaktionsrohr axial mit einer zentralen Symmetrieachse des Hohlleiters fluchtet.

5. Verfahren nach einem oder mehreren der Ansprüche 1 bis 4, bei dem die Bestrahlung des Reaktionsgemisches in einem Hohlraumresonator mit koaxialem Übergang der Mikrowellen erfolgt.

6. Verfahren nach einem oder mehreren der Ansprüche 1 bis 5, bei dem der Hohlraumresonator im E₀₁ₙ-Mode betrieben wird, wobei n eine ganze Zahl von 1 bis 200 ist.

7. Verfahren nach einem oder mehreren der Ansprüche 1 bis 6, bei dem sich im Hohlraumresonator eine stehende Welle ausbildet.

8. Verfahren nach einem oder mehreren der Ansprüche 1 bis 7, bei dem das Reaktionsgut durch die Mikrowellenbestrahlung auf Temperaturen zwischen 150 und 500 °C erhitzt wird.

9. Verfahren nach einem oder mehreren der Ansprüche 1 bis 8, bei dem die Mikrowellenbestrahlung bei Drücken oberhalb des Atmosphärendrucks erfolgt.

10. Verfahren nach einem oder mehreren der Ansprüche 1 bis 9, bei dem R¹ ein gegebenenfalls substituierter aliphatischer Kohlenwasserstoffrest mit 2 bis 30 C-Atomen ist.

11. Verfahren nach einem oder mehreren der Ansprüche 1 bis 10, bei dem R¹ ein gegebenenfalls substituierter aliphatischer Kohlenwasserstoffrest mit 2 bis 30 C-Atomen ist, der mindestens eine C=C-Doppelbindung enthält.

12. Verfahren nach einem oder mehreren der Ansprüche 1 bis 10, bei dem R¹ ein gesättigter Alkylrest mit 1, 2, 3 oder 4 C-Atomen ist.

13. Verfahren nach einem oder mehreren der Ansprüche 1 bis 11, bei dem R¹ für eine gegebenenfalls substituierte Alkenylgruppe mit 2 bis 4 Kohlenstoffatomen steht.

14. Verfahren nach einem oder mehreren der Ansprüche 1 bis 9, bei dem R¹ ein gegebenenfalls substituiertes zyklisches, durchkonjugiertes System mit (4 n + 2) π-Elektronen ist, worin n gleich 1, 2, 3, 4 oder 5 ist.

15. Verfahren nach einem oder mehreren der Ansprüche 1 bis 9, bei dem die Carbonsäure der Formel I ausgewählt ist aus Ameisensäure, Essigsäure, Propionsäure, Buttersäure, iso-Buttersäure, Pentansäure, iso-Pentansäure, Pivalinsäure, Acrylsäure, Methacrylsäure, Crotonsäure, 2,2-Dimethylacrylsäure, Maleinsäure, Fumarsäure, Itaconsäure, Zimtsäure, Methoxyzimtsäure, Bernsteinsäure, Butantetracarbonsäure, Phenylessigsäure, (2-Bromphenyl)essigsäure, (Methoxyphenyl)essigsäure, (Dimethoxyphenyl)essigsäure, 2 Phenylpropionsäure, 3-Phenylpropionsäure, 3-(4-Hydroxyphenyl)propionsäure, 4-Hydroxyphenoxyessigsäure, Hexansäure, Cyclohexansäure, Heptansäure, Octansäure, Nonansäure, Neononansäure, Decansäure, Neodecansäure, Undecansäure, Neoundecansäure, Dodecansäure, Tridecansäure, Tetradecansäure, 12-Methyltridecansäure, Pentadecansäure, 13-Methyltetradecansäure, 12 Methyltetradecansäure, Hexadecansäure, 14-Methylpentadecansäure, Heptadecansäure, 15-Methylhexadecansäure, 14-Methylhexadecansäure, Octadecansäure, Iso-Octadecansäure, Icosansäure, Docosansäure, Tetracosansäure, Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure, Dodecenylbernsteinsäure, Octadecenylbernsteinsäure, aus Baumwollsamen-, Cocos-, Erdnuss-, Färberdistel-, Mais-, Palmkern-, Raps-, Oliven-, Senfsamen, Soja-, Sonnenblumenöl, Talg-, Knochen- und Fischöl gewonnene Carbonsäuremischungen, Tallölfettsäure, Harz- und Naphthensäuren, Benzoesäure, Phthalsäure, Isophthalsäure, die Isomeren der Naphthalincarbonsäure, Pyridincarbonsäure und Naphthalindicarbonsäure, Trimellitsäure, Trimesinsäure, Pyromellitsäure und Mellitsäure, die Isomeren der Methoxybenzoesäure, Hydroxybenzoesäure, Hydroxymethylbenzoesäure, Hydroxymethoxybenzoesäure, Hydroxydimethoxybenzoesäure, Hydroxyisophthalsäure, Hydroxynaphthalincarbonsäure, Hydoxypyridincarbonsäure, Hydroxymethylpyridincarbonsäure, Hydroxychinolincarbonsäure, o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure und 3,4-Dimethylbenzoesäure.

16. Verfahren nach einem oder mehreren der Ansprüche 1 bis 15, bei dem A ausgewählt ist aus aliphatischen Resten mit 1 bis 12 Kohlenstoffatomen und aromatischen Resten mit 5 bis 12 Kohlenstoffatomen.

17. Verfahren nach einem oder mehreren der Ansprüche 1 bis 16, bei dem R² ausgewählt ist aus der Gruppe bestehend aus H, gegebenenfalls substituierten aliphatischen Resten mit 2 bis 18 Kohlenstoffatomen, gegebenenfalls substituierten C₆-C₁₂-Arylgruppen, gegebenenfalls substituierten heteroaromatische Gruppe mit 5 bis 12 Ringgliedern, oder eine Gruppe der Formel -A-X, wobei
A für einen gegebenenfalls substituierten Kohlenwasserstoffrest mit 1 bis 50 Kohlenstoffatomen und
X für eine Säuregruppe oder deren Metallsalz stehen.

18. Verfahren nach einem oder mehreren der Ansprüche 1 bis 17, bei dem X ausgewählt ist aus der Gruppe bestehend aus Carbonsäuren, Sulfonsäuren und Phosphonsäuren.

19. Verfahren nach einem oder mehreren der Ansprüche 1 bis 18, bei dem X für ein Alkali- oder Erdalkalisalz einer Säuregruppe steht.

20. Verfahren nach einem oder mehreren der Ansprüche 1 bis 19, bei dem die mindestens eine Aminogruppe tragende organische Säure der Formel (II) ausgewählt ist aus α-Aminocarbonsäuren, β-Aminosulfonsäuren, Aminomethylenphosphonsäuren und deren Metallsalzen.

21. Verfahren nach einem oder mehreren der Ansprüche 1 bis 20, bei dem Carbonsäure (I) und Aminogruppe tragende organische Säure (II) im molaren Verhältnis von 20:1 bis 1 :20, jeweils bezogen auf die Molequivalente an Carboxyl- und Aminogruppen, zur Reaktion gebracht werden.

22. Verfahren nach einem oder mehreren der Ansprüche 1 bis 21, welches in Gegenwart von basischen Katalysatoren durchgeführt wird.

## Claims

1. A continuous process for N-acylation of organic acids bearing amino groups, in which at least one carboxylic acid of the formula (I)
R¹-COOH (I)
in which R¹ is hydrogen or an optionally substituted hydrocarbyl radical having 1 to 50 carbon atoms,
is reacted with at least one organic acid which bears at least one amino group and is of the formula (II)
R²NH-A-X (II)
in which
A is an optionally substituted hydrocarbyl radical having 1 to 50 carbon atoms
X is an acid group or the metal salt thereof, and
R² is hydrogen, an optionally substituted hydrocarbyl radical having 1 to 50 carbon atoms or a group of the formula -A-X in which A and also X are each independently as defined above,
under microwave irradiation in a reaction tube whose longitudinal axis is in the direction of propagation of the microwaves from a monomode microwave applicator to give the amide, in which the reaction mixture is irradiated with microwaves in a substantially microwave-transparent reaction tube within a hollow conductor connected via waveguides to a microwave generator.

2. The process as claimed in claim 1, in which the microwave applicator is configured as a cavity resonator.

3. The process as claimed in claim 1 and/or 2, in which the microwave applicator is configured as a cavity resonator of the reflection type.

4. The process as claimed in one or more of claims 1 to 3, in which the reaction tube is aligned axially with a central axis of symmetry of the hollow conductor.

5. The process as claimed in one or more of claims 1 to 4, in which the reaction mixture is irradiated in a cavity resonator with a coaxial transition of the microwaves.

6. The process as claimed in one or more of claims 1 to 5, in which the cavity resonator is operated in E₀₁ₙ mode where n is an integer from 1 to 200.

7. The process as claimed in one or more of claims 1 to 6, in which a standing wave forms in the cavity resonator.

8. The process as claimed in one or more of claims 1 to 7, in which the reaction mixture is heated by the microwave irradiation to temperatures between 150 and 500°C.

9. The process as claimed in one or more of claims 1 to 8, in which the microwave irradiation is effected at pressures above atmospheric pressure.

10. The process as claimed in one or more of claims 1 to 9, in which R¹ is an optionally substituted aliphatic hydrocarbyl radical having 2 to 30 carbon atoms.

11. The process as claimed in one or more of claims 1 to 10, in which R¹ is an optionally substituted aliphatic hydrocarbyl radical which has 2 to 30 carbon atoms and contains at least one C=C double bond.

12. The process as claimed in one or more of claims 1 to 10, in which R¹ is a saturated alkyl radical having 1, 2, 3 or 4 carbon atoms.

13. The process as claimed in one or more of claims 1 to 11, in which R¹ is an optionally substituted alkenyl group having 2 to 4 carbon atoms.

14. The process as claimed in one or more of claims 1 to 9, in which R¹ is an optionally substituted cyclic through-conjugated system having (4n + 2) π electrons where n is 1, 2, 3, 4 or 5.

15. The process as claimed in one or more of claims 1 to 9, in which the carboxylic acid of the formula I is selected from formic acid, acetic acid, propionic acid, butyric acid, isobutyric acid, pentanoic acid, isopentanoic acid, pivalic acid, acrylic acid, methacrylic acid, crotonic acid, 2,2-dimethylacrylic acid, maleic acid, fumaric acid, itaconic acid, cinnamic acid, methoxycinnamic acid, succinic acid, butanetetracarboxylic acid, phenylacetic acid, (2-bromophenyl)acetic acid, (methoxyphenyl)acetic acid, (dimethoxyphenyl)acetic acid, 2-phenylpropionic acid, 3-phenylpropionic acid, 3-(4-hydroxyphenyl)propionic acid, 4-hydroxyphenoxyacetic acid, hexanoic acid, cyclohexanoic acid, heptanoic acid, octanoic acid, nonanoic acid, neononanoic acid, decanoic acid, neodecanoic acid, undecanoic acid, neoundecanoic acid, dodecanoic acid, tridecanoic acid, tetradecanoic acid, 12-methyltridecanoic acid, pentadecanoic acid, 13-methyltetradecanoic acid, 12-methyltetradecanoic acid, hexadecanoic acid, 14-methylpentadecanoic acid, heptadecanoic acid, 15-methylhexadecanoic acid, 14-methylhexadecanoic acid, octadecanoic acid, isooctadecanoic acid, eicosanoic acid, docosanoic acid, tetracosanoic acid, myristoleic acid, palmitoleic acid, hexadecadienoic acid, delta-9-cis-heptadecenoic acid, oleic acid, petroselic acid, vaccenic acid, linoleic acid, linolenic acid, gadoleic acid, gondoic acid, eicosadienoic acid, arachidonic acid, cetoleic acid, erucic acid, docosadienic acid and tetracosenoic acid, dodecenylsuccenic acid, octadecenylsuccenic acid, carboxylic acid mixtures obtained from cottonseed oil, coconut oil, peanut oil, safflower oil, corn oil, palm kernel oil, rapeseed oil, olive oil, mustardseed oil, soybean oil, sunflower oil, tallow oil, bone oil and fish oil, tall oil fatty acid, resin acids and naphthenic acids, benzoic acid, phthalic acid, isophthalic acid, the isomers of naphthalenecarboxylic acid, pyridinecarboxylic acid and naphthalenedicarboxylic acid, trimellitic acid, trimesic acid, pyromellitic acid and mellitic acid, the isomers of methoxybenzoic acid, hydroxybenzoic acid, hydroxymethylbenzoic acid, hydroxymethoxybenzoic acid, hydroxydimethoxybenzoic acid, hydroxyisophthalic acid, hydroxynaphthalenecarboxylic acid, hydoxypyridinecarboxylic acid, hydroxymethylpyridinecarboxylic acid, hydroxyquinolinecarboxylic acid, o-toluic acid, m-toluic acid, p-toluic acid, o-ethylbenzoic acid, m-ethylbenzoic acid, p-ethylbenzoic acid, o-propylbenzoic acid, m-propylbenzoic acid, p-propylbenzoic acid and 3,4-dimethylbenzoic acid.

16. The process as claimed in one or more of claims 1 to 15, in which A is selected from aliphatic radicals having 1 to 12 carbon atoms and aromatic radicals having 5 to 12 carbon atoms.

17. The process as claimed in one or more of claims 1 to 16, in which R² is selected from the group consisting of H, optionally substituted aliphatic radicals having 2 to 18 carbon atoms, optionally substituted C₆-C₁₂-aryl groups, optionally substituted heteroaromatic groups having 5 to 12 ring members, or a group of the formula -A-X where
A is an optionally substituted hydrocarbyl radical having 1 to 50 carbon atoms and
X is an acid group or the metal salt thereof.

18. The process as claimed in one or more of claims 1 to 17, in which X is selected from the group consisting of carboxylic acids, sulfonic acids and phosphonic acids.

19. The process as claimed in one or more of claims 1 to 18, in which X is an alkali metal or alkaline earth metal salt of an acid group.

20. The process as claimed in one or more of claims 1 to 19, in which the organic acid which bears at least one amino group and is of the formula (II) is selected from α-aminocarboxylic acids, β-aminosulfonic acids, aminomethylenephosphonic acids and metal salts thereof.

21. The process as claimed in one or more of claims 1 to 20, in which carboxylic acid (I) and organic acid (II) bearing an amino group are reacted in a molar ratio of 20:1 to 1:20, based in each case on the molar equivalents of carboxyl and amino groups.

22. The process as claimed in one or more of claims 1 to 21, which is performed in the presence of basic catalysts.

## Revendications

1. Procédé continu de N-acylation d'acides organiques portant des groupes amino, selon lequel au moins un acide carboxylique de formule (I)
R¹-COOH (I)
dans laquelle R¹ représente l'hydrogène ou un radical hydrocarboné éventuellement substitué contenant 1 à 50 atomes de carbone,
est mis en réaction avec au moins un acide organique portant au moins un groupe amino de formule (II)
R²NH-A-X (II)
dans laquelle
A représente un radical hydrocarboné éventuellement substitué contenant 1 à 50 atomes de carbone,
X représente un groupe acide ou son sel métallique et
R² représente l'hydrogène, un radical hydrocarboné éventuellement substitué contenant 1 à 50 atomes C ou un groupe de formule -A-X, dans laquelle A tout comme X présentent indépendamment l'un de l'autre les significations indiquées précédemment,
pour former l'amide sous exposition à des micro-ondes dans un tube de réaction dont l'axe longitudinal se trouve dans la direction de propagation des micro-ondes d'un applicateur de micro-ondes monomodal, dans lequel l'exposition du mélange réactionnel à des micro-ondes a lieu dans un tube de réaction essentiellement transparent aux micro-ondes à l'intérieur d'un conducteur creux raccordé avec un générateur de micro-ondes par un guide d'ondes.

2. Procédé selon la revendication 1, dans lequel l'applicateur de micro-ondes est configuré sous la forme d'une cavité résonante.

3. Procédé selon la revendication 1 et/ou 2, dans lequel l'applicateur de micro-ondes est configuré sous la forme d'une cavité résonante de type à réflexion.

4. Procédé selon une ou plusieurs des revendications 1 à 3, dans lequel le tube de réaction est aligné axialement avec un axe de symétrie central du conducteur creux.

5. Procédé selon une ou plusieurs des revendications 1 à 4, dans lequel l'exposition du mélange réactionnel a lieu dans une cavité résonante avec croisement coaxial des micro-ondes.

6. Procédé selon une ou plusieurs des revendications 1 à 5, dans lequel la cavité résonante est utilisée en mode E₀₁ₙ, n étant un nombre entier de 1 à 200.

7. Procédé selon une ou plusieurs des revendications 1 à 6, dans lequel une onde stationnaire se forme dans la cavité résonante.

8. Procédé selon une ou plusieurs des revendications 1 à 7, dans lequel le mélange réactionnel est porté à des températures comprises entre 150 et 500 °C par l'exposition à des micro-ondes.

9. Procédé selon une ou plusieurs des revendications 1 à 8, dans lequel l'exposition à des micro-ondes a lieu à des pressions supérieures à la pression atmosphérique.

10. Procédé selon une ou plusieurs des revendications 1 à 9, dans lequel R¹ représente un radical hydrocarboné aliphatique éventuellement substitué contenant 2 à 30 atomes C.

11. Procédé selon une ou plusieurs des revendications 1 à 10, dans lequel R¹ représente un radical hydrocarboné aliphatique éventuellement substitué contenant 2 à 30 atomes C, qui contient au moins une double liaison C=C.

12. Procédé selon une ou plusieurs des revendications 1 à 10, dans lequel R¹ représente un radical alkyle saturé contenant 1, 2, 3 ou 4 atomes C.

13. Procédé selon une ou plusieurs des revendications 1 à 11, dans lequel R¹ représente un groupe alcényle éventuellement substitué contenant 2 à 4 atomes de carbone.

14. Procédé selon une ou plusieurs des revendications 1 à 9, dans lequel R¹ représente un système entièrement conjugué cyclique éventuellement substitué contenant (4n + 2) électrons Π, n valant 1, 2, 3, 4 ou 5.

15. Procédé selon une ou plusieurs des revendications 1 à 9, dans lequel l'acide carboxylique de formule I est choisi parmi l'acide formique, l'acide acétique, l'acide propionique, l'acide butyrique, l'acide iso-butyrique, l'acide pentanoïque, l'acide iso-pentanoïque, l'acide pivalique, l'acide acrylique, l'acide méthacrylique, l'acide crotonique, l'acide 2,2-diméthylacrylique, l'acide maléique, l'acide fumarique, l'acide itaconique, l'acide cinnamique, l'acide méthoxycinnamique, l'acide succinique, l'acide butanetétracarboxylique, l'acide phénylacétique, l'acide (2-bromophényl)acétique, l'acide (méthoxyphényl)acétique, l'acide (diméthoxyphényl)acétique, l'acide 2-phénylpropionique, l'acide 3-phénylpropionique, l'acide 3-(4-hydroxyphényl)propionique, l'acide 4-hydroxyphénoxyacétique, l'acide hexanoïque, l'acide cyclohexanoïque, l'acide heptanoïque, l'acide octanoïque, l'acide nonanoïque, l'acide néononanoïque, l'acide décanoïque, l'acide néodécanoïque, l'acide undécanoïque, l'acide néoundécanoïque, l'acide dodécanoïque, l'acide tridécanoïque, l'acide tétradécanoïque, l'acide 12-méthyltridécanoïque, l'acide pentadécanoïque, l'acide 13-méthyltétradécanoïque, l'acide 12-méthyltétradécanoïque, l'acide hexadécanoïque, l'acide 14-méthylpentadécanoïque, l'acide heptadécanoïque, l'acide 15-méthylhexadécanoïque, l'acide 14-méthylhexadécanoïque, l'acide octadécanoïque, l'acide iso-octadécanoïque, l'acide icosanoïque, l'acide docosanoïque, l'acide tétracosanoïque, l'acide myristoléique, l'acide palmitoléique, l'acide hexadécadiénoïque, l'acide delta-9-cis-heptadécénoïque, l'acide oléique, l'acide pétrosélinique, l'acide vaccénique, l'acide linoléique, l'acide linolénique, l'acide gadoléique, l'acide gondoïque, l'acide icosadiénoïque, l'acide arachidonique, l'acide cétoléique, l'acide érucique, l'acide docosadiénoïque et l'acide tétracosénoïque, l'acide dodécénylsuccinique, l'acide octadécénylsuccinique, les mélanges d'acides carboxyliques obtenus à partir de graines de coton, de graines de coco, d'arachide, de serratule, de maïs, de palmiste, de colza, d'olive, de moutarde, d'huile de soja, de tournesol, d'huile de suif, d'os et de poisson, l'acide gras de tallöl, les acides résiniques et naphténiques, l'acide benzoïque, l'acide phtalique, l'acide isophtalique, les isomères de l'acide naphtalinecarboxylique, de l'acide pyridinecarboxylique et de l'acide naphtalinedicarboxylique, l'acide triméllitique, l'acide trimésique, l'acide pyroméllitique et l'acide méllitique, les isomères de l'acide méthoxybenzoïque, de l'acide hydroxybenzoïque, de l'acide hydroxyméthylbenzoïque, de l'acide hydroxyméthoxybenzoïque, de l'acide hydroxydiméthoxybenzoïque, de l'acide hydroxyisophtalique, de l'acide hydroxynaphtalinecarboxylique, de l'acide hydroxypyridinecarboxylique, de l'acide hydroxyméthylpyridinecarboxylique, de l'acide hydroxyquinolinecarboxylique, l'acide o-tolylique, l'acide m-tolylique, l'acide p-tolylique, l'acide o-éthylbenzoïque, l'acide m-éthylbenzoïque, l'acide p-éthylbenzoïque, l'acide o-propylbenzoïque, l'acide m-propylbenzoïque, l'acide p-propylbenzoïque et l'acide 3,4-diméthylbenzoïque.

16. Procédé selon une ou plusieurs des revendications 1 à 15, dans lequel A est choisi parmi les radicaux aliphatiques contenant 1 à 12 atomes de carbone et les radicaux aromatiques contenant 5 à 12 atomes de carbone.

17. Procédé selon une ou plusieurs des revendications 1 à 16, dans lequel R² est choisi dans le groupe constitué par H, les radicaux aliphatiques éventuellement substitués contenant 2 à 18 atomes de carbone, les groupes aryle en C₆-C₁₂ éventuellement substitués, un groupe hétéroaromatique éventuellement substitué contenant 5 à 12 éléments de cycle ou un groupe de formule -A-X, dans laquelle
A représente un radical hydrocarboné éventuellement substitué contenant 1 à 50 atomes de carbone et
X représente un groupe acide ou son sel métallique.

18. Procédé selon une ou plusieurs des revendications 1 à 17, dans lequel X est choisi dans le groupe constitué par les acides carboxyliques, les acides sulfoniques et les acides phosphoniques.

19. Procédé selon une ou plusieurs des revendications 1 à 18, dans lequel X représente un sel alcalin ou alcalino-terreux d'un groupe acide.

20. Procédé selon une ou plusieurs des revendications 1 à 19, dans lequel l'acide organique portant au moins un groupe amino de formule (II) est choisi parmi les acides α-aminocarboxyliques, les acides β-aminosulfoniques, les acides aminométhylènephosphoniques et leurs sels métalliques.

21. Procédé selon une ou plusieurs des revendications 1 à 20, dans lequel l'acide carboxylique (I) et l'acide organique portant un groupe amino (II) sont mis en réaction en un rapport molaire de 20:1 à 1:20, à chaque fois par rapport aux équivalents molaires de groupes carboxyle et amino.

22. Procédé selon une ou plusieurs des revendications 1 à 21, qui est réalisé en présence de catalyseurs basiques.
